# EUROPÄISCHE PATENTSCHRIFT

(11) **EP 1 123 092 B1**
(45) Veröffentlichungstag und Bekanntmachung des Hinweises auf die Patenterteilung: **28.08.2002**
(21) Anmeldenummer: 99947484.4
(22) Anmeldetag: 08.10.1999
(51) Int. Cl.: A61K 31/225, A61K 31/28, A61K 9/16, A61K 9/50, A61P 1/00, A61P 17/00, A61P 17/06

(54) **FUMARSÄURE-MIKROTABLETTEN**
FUMARIC ACID MICROTABLETS
MICROCOMPRIMES D'ACIDE FUMARIQUE

(30) Priorität: 20.10.1998 DE 19848260
(43) Veröffentlichungstag der Anmeldung: 16.08.2001
(73) Patentinhaber: Fumapharm AG, 5630 Muri (CH)
(72) Erfinder: JOSHI, Rajendra, Kumar, CH-8048 Zürich (CH); STREBEL, Hans-Peter, CH-5630 Muri (CH)
(74) Vertreter: Teipel, Susanne, Dr.
(86) Internationale Anmeldenummer: EP9907568
(87) Internationale Veröffentlichungsnummer: WO00023068

(56) Entgegenhaltungen:
- DE-A- 3 834 794
- US-A- 5 424 332

## Beschreibung

Die Erfindung betrifft die Verwendung bestimmter Fumarsäuremonoalkylestersalze allein oder in Verbindung mit einem Dialkylfumarat zur Herstellung von Mikrotabletten zur Behandlung der psoriatischen Arthrits, Neurodermitis, Psoriasis und Enteritis regionalis Crohn.

In der europäischen Patentanmeldung EP-A-0 188 749 sind bereits Fumarsäurederivate und diese enthaltende pharmazeutische Zubereitungen zur Behandlung der Psoriasis beschrieben. Ebenso sind aus der DE-A-25 30 372 pharmazeutische Zubereitungen zur Behandlung der Psoriasis bekannt, die eine Mischung aus Fumarsäure und weiteren Fumarsäurederivaten enthalten. Ein Anteil freier Fumarsäure ist hier obligatorisch.

Weiterhin beschreibt die DE-A-26 21 214 Arzneimittel zur Behandlung von der Psoriasis, die Fumarsäuremonoethylester und dessen Mineralsalze als Wirkstoff enthalten. Ebenso ist aus der Publikation (Hautarzt 1987) S. 279 bis 285 die Verwendung von Fumarsäuremonoethylestersalzen des Calciums, Zinks und Magnesiums sowie von Fumarsäuredimethylester für die Psoriasisbehandlung bekannt.

Schließlich offenbart die EP 0 312 697 pharmazeutische Zubereitungen, die eine oder mehrere Verbindungen, ausgewählt unter den Calcium-, Magnesium-, Zink- und Eisensalzen des Fumarsäuremonomethylesters, allein oder vorzugsweise im Gemisch mit C₁₋₅-Alkylfumaraten enthalten. Ein Präparat gemäß Beispiel 4 dieser Druckschrift enthält 87,5 mg Monoethylfumarat Ca-Salze, 120,0 mg Dimethylfumarat, 5,0 mg Monoethylfumarat Mg-Salz und 3,0 mg Monoethylfumarat Zn-Salz, entsprechend 164 mg Fumarsäure. Das Präparat liegt in Form von magensaftresitenten Tabletten vor und ist in Deutschland unter dem Markennamen Fumaderm® in Deutschland zugelassen und auf dem Markt erhältlich.

Bereits während der Phase 3 der klinischen Untersuchungen und bei den Postmarketing-Studien zu diesem Präparat wurde festgestellt, daß etwa 60 % der Patienten während der Initalphase mit Fumaderm® über gastro-intestinale Beschwerden in Form von Durchfall, Magenschmerzen und Völlegefühl klagten. Als weitere Nebenwirkung treten außerdem häufig der sogenannte Flush, also Gesichtsrötungen und Hitzegefühle auf.

Zwar werden die Tabletten in der Regel relativ gut vertragen, jedoch treten vor allem zu Beginn der Therapie die genannten Beschwerden immer wieder auf. Im Laufe der Behandlung gehen die unerwünschten Nebenwirkungen häufig zurück. Es gibt jedoch Patienten, bei denen die Einnahme von Fumaderm® schwere gastro-intestinale Beschwerden verursacht. Die auftretenden Magen-, Darmbeschwerden verringern die Patientenkompliance und können für den Patienten so beeinträchtigend sein, daß sie zum Teil einen Grund für einen Therapieabbruch darstellen.

Es war daher Aufgabe der vorliegenden Erfindung, eine pharmazeutische Zubereitung bereitzustellen, mittels derer die genannten Nebenwirkungen, insbesondere die gastro-intestinalen Beschwerden vermieden werden können, bei gleichzeitiger Verabreichung derselben pharmazeutischen Inhaltsstoffe.

Diesbezügliche Untersuchungen der Anmelderin haben gezeigt, daß Methylhydrogenfumarat ein Metabolit des Dimethylfumarats, welches den Hauptbestandteil des Präparates Fumaderm® darstellt, die Endotoxin-stimulierte TNF-alpha Sekretion in menschlichen mononuclearen Zellen des Peripheriebluts (periphere blood mononuclear cells = PBMC-Zellen) und in isolierten Monozyten initial steigert. Bei multipler Reexposition kommt es zur Abnahme der Endotoxin-induzierten TNF-alpha Sekretionssteigerung, also einem Ge wöhnungseffekt.

Möglicherweise ist diese initiale Induktion von TNF-alpha für die bekannte Nebenwirkung des Präparats Fumaderm® wie die gastro-intestinalen Beschwerden oder die Flush-Symptomatic verantwortlich. Die Tendenz zur Abnahme der Endotoxin-induzierten TNF-alpha Sekretion nach wiederholter Methylhydrogenfumarat-Exposition kann eine Erklärung für den Gewöhnungseffekt, d.h. das Nachlassen der Nebenwirkungen bei andauernder Fumaderm®-Behandlung sein. Dementsprechend war es zunächst Ziel weiterer Untersuchungen, die TNF-alpha Sekretion durch andere Medikamente zu hemmen und dadurch die Nebenwirkungen der Verabreichung von Fumaderm zu kontrollieren.

Überraschender- und unerwarteter Weise stellte sich im Rahmen dieser Untersuchungen heraus, daß eine Formulierung der Wirkstoffe in Form von Mikrotabletten die gastro-intestinalen Beschwerden wesentlich reduzieren konnte. Erfindungsgemäß wird die obige Aufgabe daher durch Verwendung eines oder mehrerer Fumarsäuremonoalkylestersalze der allgemeinen Formel 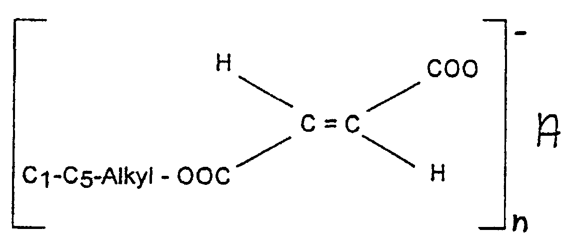 gegebenenfalls im Gemisch mit Dialkylfumarat der Formel 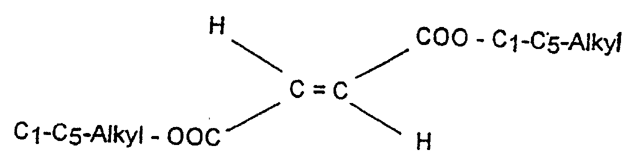 wobei A ein zweiwertiges Kation der Reihe Ca, Mg, Zn oder Fe bzw. ein einwertiges Kation aus der Reihe Li, Na oder K und n die Zahl 1 oder 2 je nach Art des Kations bedeuten, und gegebenenfalls üblicher pharmazeutischer Hilfs- und Trägerstoffe zur Herstellung einer pharmazeutischen Zubereitung in Form von Mikrotabletten oder Mikropellets zur Behandlung der psoriatischen Arthritis, Neurodermitits, Psoriasis und Enteritis regionalis Crohn.

Vorzugsweise liegt die Größe bzw. der mittlere Durchmesser der Mikropellets oder Mikrotabletten im Bereich von 300 bis 2.000 µm insbesondere im Bereich von 500 bis 1.500 µm und am meisten bevorzugt bei 1.000 µm.

Die Mikrotabletten oder -Pellets können in Kapsein oder Sachets gefüllt und in dieser Form verabreicht werden. Weiterhin können die Mikrotabletten selbst oder die Kapseln mit einem magensaftresistenten Überzug versehen sein. Dieser Überzug wird gemäß herkömmlicher Verfahren aufgebracht. Bei den Kapseln kann es sich um Hart- oder Weichgelantine-Kapseln handeln.

Bevorzugte Zubereitungen gemäß der Erfindungen enthalten das Calciumsalz des Fumarsäuremonomethylesters und/oder das Calciumsalz des Fumarsäuremonoethylesters, gegebenenfalls im Gemisch mit Dimethylfumarat. Das Gesamtgewicht der Wirkstoffe beträgt hierbei 10 bis 300 mg. Vorzugsweise enthält die Zubereitung in Form von Mikrotabletten 10 bis 290 Gewichtsteile des Fumarsäuremonoalkylesters (Calciumsalz) und 290 bis 10 Gewichtsteile Dimethylfumarat. Gemäß einer weiteren Ausführungsform kann diese Zubereitung außerdem 1 bis 50 Gewichtsteile des Zinksalzes Fumarsäuremonoalkylesters enthalten.

Eine weitere bevorzugte Zubereitung in Form von Mikrotabletten enthält 10 bis 250 Gewichtsteile des Fumarsäuremonoalkylesters (Calciumsalz), 250 bis 10 Gewichtsteile Dimethylfumarat, 1 bis 50 Gewichtsteile Fumarsäuremonoalkylester (Magnesiumsalz) und 1 bis 50 Gewichtsteile Fumarsäuremonoalkylester (Zinksalz), wobei das Gesamtgewicht der Wirkstoffe 30 bis 300 mg beträgt.

Weitere bevorzugte Zubereitungen werden in den Ansprüchen beschrieben.

Für den systemischen Einstieg in die Behandlung wie auch umgekehrt für den Ausstieg (ausschleichende Dosierung) ist eine niedrige Dosierung vorteilhaft. Diese kann sich bspw. aus 30 mg Dimethylfumarat, 20 mg Monoethylfumarat (Calciumsalz) und 3 mg Monoethylfumarat bzw. Monomethylfumarat (Zinksalze) zusammensetzen. Für die therapeutische Dosierung nach einer Einstiegsphase kann bspw. eine Dosierung von 20 mg Dimethylfumarat, 87 mg Monoethylfumarat (Calciumsalz) und 3,0 mg Monoethylfumarat bzw. Monomethylfumarat (Zinksalze) zur Anwendung kommen.

Die erfindungsgemäß verwendeten Fumarsäurederivate werden bspw. gemäß den in der EP 0 312 697 beschriebenen Verfahren erhalten.

Ohne sich durch theoretische Überlegungen binden lassen zu wollen, wird vermutet, daß die gastro-intestinalen Beschwerden durch lokale Reize an den Darmepithelzellen verursacht sein könnten, welche die TNF-alpha-Sékretion induziert. Vermutlich werden bei Verabreichung herkömmlicher Tabletten die Inhaltsstoffe der Tablette lokal in zu hoher Konzentration im Darm freigesetzt, wodurch die örtliche Reizung der Darmschleimhaut verursacht wird. Durch diesen lokalen Reiz werden kurzfristig sehr hohe Konzentrationen an TNF-alpha freigesetzt, welche für die gastro-intestinalen Nebenwirkungen verantwortlich sein könnten. Bei der Applikation von magensaftresistenten Mikrotabletten in Kapseln werden dagegen lokal niedrige Konzentrationen der Wirkstoffe an den Darmepithellzellen erzielt. Die Mikrotabletten werden vom Magen nach und nach durch die Magenperistaltik in den Dünndarm abgegeben und es kommt zu einer besseren Verteilung der Wirkstoffe.

Magensaftresistente Mikrotabletten der gleichen Dosierung verteilen sich also bereits im Magen und gelangen daher bereits portionsweise in den Darm, wo die Wirkstoffe in kleineren Dosen freigesetzt werden. So wird die lokale Reizung der Darmepithelzellen vermieden, ebenso wie die Freisetzung von TNF-alpha. Hieraus resultiert vermutlich die bessere Magen-Darmverträglichkeit von Mikrotabletten gegenüber herkömmlichen Tabletten. Daß lediglich die Veränderung der Galenik zu einer derart drastischen Reduktion der Nebenwirkungen führen würde, konnte jedoch nicht erwartet werden.

Die Herstellung und Wirkungsweise der erfindungsgemäßen Mikrotabletten soll anhand der folgenden Beispiele verdeutlicht werden:

### Herstellungsbeispiele:

### Beispiel 1:

Herstellung von magensaftresistenten Mikrotabletten in Kapseln, enthaltend 87,0 mg Monoethylfumarat Ca-Salz, 120,0 mg Dimethylfumarat, 5,0 mg Monoethylfumarat Mg-Salz, entsprechend insgesamt 164 mg Fumarsäure

8,700 kg Monoethylfumarat Ca-Salz, 12,000 kg Dimethylfumarat, 0,500 kg Monoethylfumarat Mg-Salz und 0,30 kg Monoethylfumarat Zn-Salz werden zerkleinert, intensiv gemischt und mittels eines Siebes 800 unter entsprechenden Vorsichtsmaßnahmen (Atemmaske, Handschuhe, Schutzanzug, etc.) homogenisiert. Es wird ein Hilfstoffgemisch folgender Zusammensetzung hergestellt: 18,00 kg Stärkederivat (STA-RX® 1500), 0,30 kg Cellulose mikrokristallin (Avicel® PH 101), 0,75 kg PVP (Kollidon® 120), 4,00 kg Primogel®, 0,25 kg Kieselsäure kolloidal (Aerosil®). Das gesamte Pulvergemisch wird mit einem Wirkstoffgemisch versetzt, mittels eines Siebes 200 homogenisiert, mit einer 2%-igen wäßrigen Lösung von Polyvinylpyrrolidon (Kollidon® K25) auf übliche Weise zu einem Bindemittelgranulat verarbeitet und in trockenem Zustand mit der äußeren Phase gemischt. Diese besteht aus 0,50 kg Mg-Stearat und 1,50 kg Talkum. Das Pulvergemisch wird anschließend auf übliche Weise zu gewölbten Mikrotabletten von 10,0 mg Bruttomasse und 2,0 mm Durchmesser gepreßt. Anstelle dieser klassischen Tablettiermethode können auch andere Methoden zur Herstellung von Tabletten verwendet werden, wie die Direkttablettierung sowie Verfahren zu Herstellung fester Dispersionen nach der Schmelzmethode und die Sprühtrocknungsmethode.

Der magensaftresistente Überzug kann in einem klassischen Dragierkessel aufgeleert oder aufgesprüht sowie in einer Wirbelschichtapparatur erfolgen. Zum Erreichen der Magensaftresistenz wird hier bspw. portionsweise eine Lösung von 2,250 kg Hydroxypropylmethylcellulosephthalat (HPMCP, Pharmacoat® HP 50), in einem Gemisch folgender Lösungsmittel aufgelöst: Aceton 13,00 1, Ethanol (94 Gew.-% denaturiert mit 2 % Keton) 13,50 1 und Aqua demineralisata 1,50 1. Zu der fertigen Lösung wird als Weichmacher Rizinusöl (0,240 kg) zugegeben und auf übliche Weise in Portionen auf die Tablettenkerne aufgetragen.

Nach beendeter Trocknung wird anschließend in der gleichen Apparatur eine Suspension folgender Zusammensetzung als Filmcoat aufgetragen: Talk 0,340 kg, Titan-(VI)-oxid Cronus RN 56 0,400 kg, Farblack L-Rotlack 86837 0,324 kg, Eudragit E 12,5 % 4,800 kg und Polyethylenglycol 6000 pH 11 XI 0,120 kg in einem Lösungsmittelgemisch folgender Zusammensetzung: 2-Propanol 8,170 kg, Aqua demineralisata 0,200 kg und Glycerintriacetat (Triacetin) 0,600 kg.

Die magensaftresistenten Mikrotabletten werden anschließend in Hartgelatine-Steckkapseln zu 500,0 mg Nettogewicht eingefüllt und verschlossen.

### Beispiel 2:

Herstellung von magensaftresistenten Mikrotabletten in Kapseln, enthaltend 87,0 mg Monoethylfumarat Ca-Salz, 120,0 mg Dimethylfumarat, 5,0 mg Monoethylfumarat Mg-Salz, entsprechend insgesamt 164 mg Famarsäure

8,700 kg Monoethylfumarat Ca-Salz, 12,000 kg Dimethylfumarat, 0,500 kg Monoethylfumarat Mg-Salz und 0,30 kg Monoethylfumarat Zn-Salz werden zerkleinert, intensiv gemischt und mittels eines Siebes 800 unter entsprechenden Vorsichtsmaßnahmen (Atemmaske, Handschuhe, Schutzanzug, etc.) homogenisiert. Es wird ein Hilfstoffgemisch folgender Zusammensetzung hergestellt: 24,70 kg mikrokristalline Cellulose (Avicel® PH 200), 3,00 kg Croscarmellose Natrium (AC-Di-SOL-SD-711), 2,50 kg Talkum, 0,10 kg Siliciumdioxid wasserfrei (Aerosil® 200) und 1,00 kg Magnesiumstearat. Das gesamte Hilfsstoffgemisch wird mit dem Wirkstoffgemisch versetzt und homogen gemischt. Das Pulvergemisch wird anschließend mittels Direkttablettierung zu gewölbten Mikrotabletten von 10,0 mg Bruttomasse und 2,0 mm Durchmesser gepreßt. Anstelle dieser klassischen Tablettiermethode können auch andere Methoden zur Herstellung von Tabletten verwendet werden, wie feste Dispersionen nach der Schmelzmethode und die Sprühtrocknungsmethode oder Tablettierung von Bindemittelgranulaten.

Der magensaftresistente Überzug kann in einem klassischen Dragierkessel aufgeleert oder aufgesprüht sowie in einer Wirbelschichtapparatur erfolgen. Hier wird bspw. eine Lösung von 0,94 kg Eudragit® L in Isopropanol hergestellt, die zusätzlich 0,07 kg Dibutylphthalat enthält. Diese Lösung wird auf die Tablettenkerne aufgesprüht.

Danach wird eine Dispersion von 17,32 kg Eudragit® L D-55 und einer Mischung aus 2,80 kg Mikrotalkum, 2,00 kg Macrogol 6000 und 0,07 kg Dimetican in Wasser hergestellt und auf die Kerne aufgesprüht.

Die magensaftresistenten Mikrotabletten werden anschließend in Hartgelatine-Steckkapseln zu 760,0 mg Nettogewicht eingefüllt und verschlossen.

### Behandlungsbeispiele

Gemäß den obigen Herstellungsbeispielen wurden Mikrotabletten mit denselben 4 Wirkstoffen in der gleichen quantitativen Zusammensetzung, wie sie das Handelsprodukt Fumaderm® enthält, hergestellt. Eine magensaftresistente Tablette Fumaderm® entspricht etwa 102 magensaftresistenten Mikrotabletten derselben Zusammensetzung. Diese Mikrotabletten werden, wie in den Herstellungsbespielen beschrieben, zur praktischeren Verabreichung in Kapseln gefüllt. Zwei Kapseln entsprechen einer Tablette Fumaderm®.

Zum besseren Vergleich wurden 2 Patienten, welche an schweren Magen-Darm-Beschwerden unter der Behandlung mit Fumaderm®-Tabletten litten, mit den magensaftesistenten Mikrotabletten gemäß der vorliegenden Erfindung behandelt. Überraschenderweise klagten diese Patienten nach Verabreichung der Mikrotabletten nicht mehr über die bei Verabreichung der herkömmlichen Tablette beobachteten gastro-intestinalen Beschwerden. Die Verbesserung der Psoriasis verhielt sich wie unter Therapie mit den Fumaderm®-Tabletten des Standes der Technik, wobei unter Umständen eine geringere Dosis bei Verabreichung in Form von Mikrotabletten ausreicht, um einen klinischen Erfolg zu erzielen.

Die Behandlungsergebnisse sind in der folgenden Tablelle dargestellt:

| | Patient 1 | Patient 2 | Produkt* |
|---|---|---|---|
| Initialien | M.M. | W.F. | |
| Alter | 63 | 54 | |
| Geschlecht | weiblich | männlich | |
| Dosierung | 14.01.1998 bis 01.04.1998 Fumaderm initial | 1985 3 Tabletten Fumaderm/Tag | Fumaderm® initial / Fumaderm® |
| GI-Beschwerden | Krämpfe, Schmerzen | Oberbauchschmerzen | " |
| Schweregrad bei GI-Beschwerden | schwer | schwer | " |
| Klinischer Befund der Psoriasis | befriedigend | befriedigend | " |
| Behandlungspause | keine | 1985 bis 12.05.1998 | " |
| Dosierung | 01.04. bis 06.04.1998 3 Kapseln/Tag 07.04 bis 10.05.1998 9 Kapseln/Tag 11.05 bis 31.08.1998 3 Kapseln/Tag | 13.05. bis 20.05.1998 3 Kapseln/Tag 21.05. bis 01.07.1998 6 Kapseln/Tag | Fumaderm® P mikro |
| GI-Beschwerden | keine | 15.05. bis 18.05 1998 Blähungen | " |
| Schweregrad der GI-Beschwerden | ― | leicht | " |
| Klinischer Befund der Psoriasis | sehr gut | gut | " |

| | | | |
|---|---|---|---|
| * Eine Fumaderm® Tablette entspricht zwei Kapseln Fumaderm P mikro GI = gastro intestinal | | | |

Aus der Tabelle ist ersichtlich, daß die Verabreichung von Mikrotabletten sogar in erhöhter Dosis (9 Kapseln täglich) keine oder nur leichte Nebenwirkungen verursachte, während bereits die niedrigere Dosierung des Handelspräparates Fumaderm® zu schweren gastro-intestinalen Beschwerden führte.

Ebenfalls aus den Behandlungsergebnissen ersichtlich ist, daß die Mikrotabletten hinsichtlich ihrer Wirksamkeit zur Behandlung der Psoriasis mit dem Handelspräparat mindestens identisch, wenn nicht verbesserte Wirkung aufweisen. Insgesamt zeigen daher die erfindungsgemäße Formulierung von Fumarsäurederivaten in Form von Mikrotabletten eine deutliche Verbesserung gegenüber der Therapie mit herkömmlichen Tabletten.

## Patentansprüche

1. Verwendung eines oder mehrerer Fumarsäuremonoalkylestersalze der allgemeinen Formel 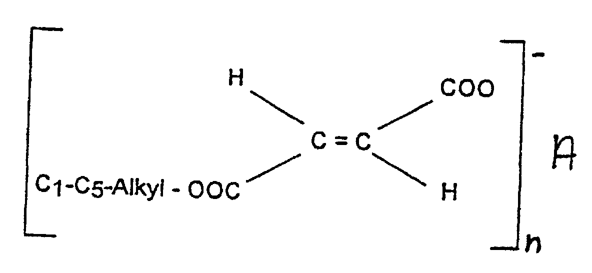 gegebenenfalls im Gemisch mit Dialkylfumarat der Formel 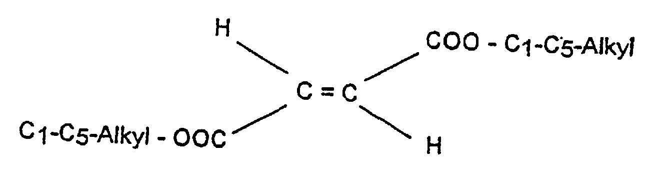 wobei A ein zweiwertiges Kation der Reihe Ca, Mg, Zn oder Fe bzw. ein einwertiges Kation aus der Reihe Li, Na oder K und n die Zahl 1 oder 2 je nach Art des Kations bedeuten, und gegebenenfalls üblicher pharmazeutischer Hilfsund Trägerstoffe zur Herstellung einer pharmazeutischen Zubereitung in Form von Mikrotabletten oder Mikropellets zur Behandlung der psoriatischen Arthritis, Neurodermitits, Psoriasis und Enteritis regionalis Crohn.

2. Verwendung nach Anspruch 1, **dadurch gekennzeichnet, daß** es sich um das Calciumsalz des Fumarsäuremonoethylesters oder -monomethylesters handelt.

3. Verwendung nach Anspruch 1 oder 2, **dadurch gekennzeichnet, daß** es sich um das Calciumsalz des Fumarsäuremonoalkylesters im Gemisch mit Dimethylfumarat handelt.

4. Verwendung nach Anspruch 1, **dadurch gekennzeichnet, daß** es sich um das Calcium- und Zinksalz des Fumarsäuremonoalkylesters im Gemisch mit Dimethylfumarat handelt.

5. Verwendung nach Anspruch 1, **dadurch gekennzeichnet, daß** es sich um das Calcium-, Magnesium- und Zinksalzes des Fumarsäuremonoethylesters im Gemisch mit Dimethylfumarat handelt.

6. Verwendung nach einem der Ansprüche 1 oder 2, **dadurch gekennzeichnet, daß** es sich um das Calciumsalz des Fumarsäuremonoalkylesters in einer Menge von 10 mg bis 300 mg handelt, wobei das Gesamtgewicht der Wirkstoffe 10 bis 300 mg beträgt.

7. Verwendung nach Anspruch 3, **dadurch gekennzeichnet, daß** es sich um 10 bis 290 Gewichtsteile des Calciumsalzes des Fumarsäuremonoalkylesters und 290 bis 10 Gewichtsteile Dimethylfumarat handelt, wobei das Gesamtgewicht der Wirkstoffe 20 bis 300 mg beträgt.

8. Verwendung nach Anspruch 4, **dadurch gekennzeichnet, daß** es sich um 10 bis 250 Gewichtsteile des Calciumsalzes des Fumarsäuremonoalkylesters, 1 bis 50 Gewichtsteile Dimethylfumarat und 1 bis 50 Gewichtsteile des Zinksalzes des Fumarsäuremonoalkylesters handelt, wobei das Gesamtgewicht der Wirkstoffe 20 bis 300 mg beträgt.

9. Verwendung nach einem der Ansprüche 1 bis 5, **dadurch gekennzeichnet, daß** es sich um 10 bis 250 Gewichtsteile des Calciumsalzes des Fumarsäuremonoalkylesters, 250 bis 10 Gewichtsteile Dimethylfumarat, 1 bis 50 Gewichtsteile des Magnesiumsalzes des Fumarsäuremonoalkylesters und 1 bis 50 Gewichtsteile des Zinksalzes des Fumarsäuremonoalkylesters handelt, wobei das Gesamtgewicht der Wirkstoffe 30 bis 300 mg beträgt.

10. Verwendung gemäß einem der vorstehenden Ansprüche, bei der die Mikrotabletten oder Mikropellets mit einem magensaftresistenten Überzug versehen sind.

11. Verwendung gemäß einem der vorstehenden Ansprüche, bei der die Mikrotabletten oder Mikropellets in Kapseln oder Sachets gefüllt werden.

## Claims

1. The use of one or more salts of fumaric acid monoalkyl esters of the general formula 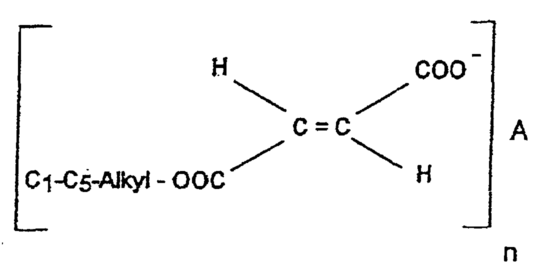 optionally in admixture with dialkyl fumarate of the formula 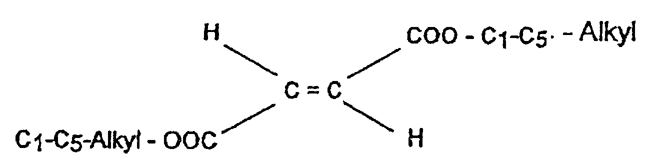 wherein A is a bivalent cation from the series consisting of Ca, Mg, Zn or Fe or a monovalent cation from the series Li, Na or K, respectively, and n denotes the numeral 1 or 2 depending on the type of cation, and, optionally, commonly used pharmaceutical excipients and vehicles for preparing a pharmaceutical composition in the form of micro-tablets or micro-pellets for treatment of psoriatic arthritis, neurodermatitis, psoriasis and enteritis regionalis Crohn.

2. The use according to claim 1, **characterised in that** the calcium salt of fumaric acid monoethyl ester or monomethyl ester is used.

3. The use according to claim 1 or 2, **characterised in that** the calcium salt of the fumaric acid monoalkyl ester is used in admixture with dimethyl fumarate.

4. The use according to claim 1, **characterised in that** the calcium and zinc salt of the fumaric acid monoalkyl ester in admixture with dimethyl fumarate is used.

5. The use according to claim 1, **characterised in that** the calcium, magnesium and zinc salt of the fumaric monoethyl ester in admixture with dimethyl fumarate is used.

6. The use according to any of the claims 1 or 2, **characterised in that** the calcium salt of the fumaric acid monoalkyl ester is used in an amount of 10 to 300 mg, the total weight of the active ingredients being 10 to 300 mg.

7. The use according to claim 3, **characterised in that** 10 to 290 parts by weight of the calcium salt of the fumaric acid monoalkyl ester and 290 to 10 parts by weight of dimethyl fumarate are used, the total weight of the active ingredients being 20 to 300 mg.

8. The use according to claim 4, **characterised in that** 10 to 250 parts by weight of the calcium salt of the fumaric acid monoalkyl ester, 1 to 50 parts by weight dimethyl fumarate and 1 to 50 parts by weight of the zinc salt of the fumaric acid mono alkyl ester are used, the total weight of the active ingredients being 20 to 300 mg.

9. The use according to any of the claims 1 to 5, **characterised in that** 10 to 250 parts by weight of the calcium salt of the fumaric acid monoalkyl ester, 250 to 10 parts by weight dimethyl fumarate, 1 to 50 parts by weight of the magnesium salt of the fumaric acid monoalkyl ester and 1 to 50 parts by weight of the zinc salt of the fumaric acid monoalkyl ester are used, the total weight of the active ingredients being 30 to 300 mg.

10. The use according to any of the previous claims, wherein the micro-tablets or micro-pellets are provided with an enteric coating (coating resistant to gastric acid).

11. The use according to any of the previous claims, wherein the micro-tablets or micro-pellets are filled into capsules or sachets.

## Revendications

1. Utilisation d'un ou de plusieurs sels de fumarates de monoalkyle de formule générale 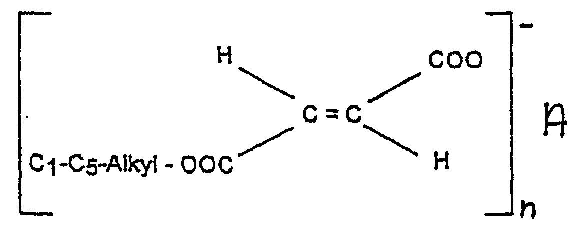 éventuellement en mélange avec du fumarate de dialkyle de formule 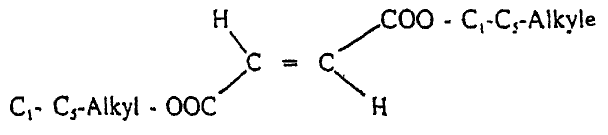 dans lesquelles A représente un cation divalent de la série Ca, Mg, Zn ou Fe ou un cation monovalent de la série Li, Na ou K et n représente le nombre 1 ou 2 selon le type du cation, et éventuellement des adjuvants et supports pharmaceutiques usuels pour la fabrication d'une préparation pharmaceutique sous la forme de microcomprimés ou de micropastilles pour le traitement de l'arthrite psoriasique, de la neurodermite et de l'entérite régionale de Crohn.

2. Utilisation selon la revendication 1, **caractérisée en ce qu'**il s'agit du sel de calcium du fumarate de monoéthyle ou de monométhyle.

3. Utilisation selon la revendication 1 ou 2, **caractérisée en ce qu'**il s'agit du sel de calcium du fumarate de monoalkyle en mélange avec le fumarate de diméthyle.

4. Utilisation selon la revendication 1, **caractérisée en ce qu'**il s'agit du sel de calcium et de zinc du fumarate de monoalkyle en mélange avec le fumarate de diméthyle.

5. Utilisation selon la revendication 1, **caractérisée en ce qu'**il s'agit du sel de calcium, de magnésium et de zinc du fumarate de monoéthyle en mélange avec le fumarate de diméthyle.

6. Utilisation selon l'une des revendications 1 ou 2, **caractérisée en ce qu'**il s'agit du sel de calcium du fumarate de monoalkyle en une quantité de 10 mg à 300 mg, la masse totale des substances actives étant de 10 à 300 mg.

7. Utilisation selon la revendication 3, **caractérisée en ce qu'**il s'agit de 10 à 290 parties en poids du sel de calcium du fumarate de monoalkyle et de 290 à 10 parties en poids de fumarate de diméthyle, la masse totale des substances actives étant de 20 à 300 mg.

8. Utilisation selon la revendication 4, **caractérisée en ce qu'**il s'agit de 10 à 250 parties en poids du sel de calcium du fumarate de monoalkyle, de 1 à 50 parties en poids de fumarate de diméthyle et de 1 à 50 parties en poids du sel de zinc du fumarate de monoalkyle, la masse totale des substances actives étant de 20 à 300 mg.

9. Utilisation selon l'une des revendications 1 à 5, **caractérisée en ce qu'**il s'agit de 10 à 250 parties en poids du sel de calcium du fumarate de monoalkyle, de 250 à 10 parties en poids de fumarate de diméthyle, de 1 à 50 parties en poids du sel de magnésium du fumarate de monoalkyle et de 1 à 50 parties en poids du sel de zinc du fumarate de monoalkyle, la masse totale des substances actives étant de 30 à 300 mg.

10. Utilisation selon l'une des revendications précédentes, dans laquelle les microcomprimés ou micropastilles sont munies d'un revêtement résistant au suc gastrique.

11. Utilisation selon l'une des revendications précédentes, dans laquelle les microcomprimés ou les micropastilles sont disposés dans des capsules ou des sachets.
